# EUROPÄISCHE PATENTSCHRIFT

(11) **EP 1 682 027 B1**
(45) Veröffentlichungstag und Bekanntmachung des Hinweises auf die Patenterteilung: **18.05.2011**
(21) Anmeldenummer: 04791027.8
(22) Anmeldetag: 29.10.2004
(51) Int. Cl.: A61B 19/02

(54) **BEHÄLTER ZUR AUFNAHME VON STERILGUT UND STERILGUT-DISPENSER**
CONTAINER FOR RECEIVING STERILE PRODUCTS AND STERILE PRODUCT DISPENSER
CONTENANT DESTINE A RECEVOIR DES ARTICLES STERILES ET DISTRIBUTEUR D'ARTICLES STERILES

(30) Priorität: 30.10.2003 DE 10350720
(43) Veröffentlichungstag der Anmeldung: 26.07.2006
(73) Patentinhaber: Grandy, Elmar, 81245 München (DE)
(72) Erfinder: Grandy, Elmar, 81245 München (DE)
(74) Vertreter: Hertz, Oliver
(86) Internationale Anmeldenummer: PCT/EP2004/012267
(87) Internationale Veröffentlichungsnummer: WO 2005/041803

(56) Entgegenhaltungen:
- EP-A- 1 090 591
- US-A- 2 835 377
- US-A- 2 985 285
- US-A- 5 554 125

## Beschreibung

Die Erfindung betrifft einen Behälter zur Aufnahme von Sterilgut mit den Merkmalen gemäß dem Oberbegriff von Anspruch 1. Die Erfindung betrifft insbesondere einen Behälter für Akupunkturnadeln. Die Erfindung betrifft des Weiteren einen Sterilgut-Dispenser und Verfahren zur Bereitstellung von Sterilgut, wie insbesondere von Akupunkturnadeln, unter Verwendung eines Sterilgut-Dispensers.

In zahlreichen Bereichen der Medizin, Biologie und Biochemie besteht ein Interesse an sterilen Arbeitsbedingungen, beispielsweise bei Eingriffen am lebenden Gewebe, bei Manipulationen oder Messungen an Zellen oder Zellkulturen oder dergleichen. Häufig werden technische Hilfsmittel, wie z.B. Spritzen oder Skalpelle, oder Hilfsmaterialien, wie z.B. Implantatmaterialien verwendet, die unter sterilen Bedingungen in einem schützenden Behälter gelagert und erst unmittelbar bei der Anwendung freigegeben werden.

Häufig werden als Sterilgut-Behälter Blisterverpackungen verwendet, die beispielsweise aus einer Kunststoff-Formschale und einer Versiegelungsfolie bestehen. In Blisterverpackungen werden beispielsweise Kanülen für Spritzen oder Akupunkturnadeln steril gelagert. Blisterverpackungen besitzen die folgenden Nachteile. Erstens ist das Verpackungsmaterial in der Regel nur einmalig verwendbar. Es entsteht unerwünschter Verpackungsabfall. Zweitens ist die Entnahme des steril verpackten Materials aus der Blisterverpackung mit einem hohen manuellen Aufwand und der Gefahr verbunden, das Sterilgut unbeabsichtigt zu beschädigen oder zu verunreinigen. Schließlich besitzen Blisterverpackungen ein ungünstiges Verhältnis der Verpackungsgröße zur Größe des verpackten Sterilguts. Die Packungsdichte des Sterilguts kann nicht ohne weiteres vergrößert werden.

Aus der US 2,985,285 ist ein Magazin für eine Vielzahl von Spritzennadeln bekannt, welches für eine wiederholte Verwendung sterilisierbar ist. Für die Benutzung sind einzelne Spritzennadeln jeweils in abgetrennten Kammern angeordnet, welche jeweils mit einem entlang einer Schiene beweglichen Deckel verschlossen sind. Zur Entnahme einer Spritzennadel wird der entsprechende Deckel zur Öffnung der Kammer verschoben. Durch ihre Positionierung dienen die Deckel der einzelnen Kammern auch als Indikator, ob die jeweilige Spritzennadel bereits entnommen wurde.

US 2,835,377 offenbart einen sterilisierbaren Behälter für eine Vielzahl von Spritzennadeln, welche in verschiedenen parallelen Bohrungen angeordnet sind. Ein gemeinsamer Deckel aller Bohrungen ist so einstellbar, dass eine kleinere Öffnung darin jeweils zu einer Bohrung ausgerichtet ist, um eine in der Bohrung angeordnete Spritzennadel freizugeben.

In DE 200 15 353 U1 wird ein Behälter für Akupunkturnadeln für eine Handakupunkturvorrichtung beschrieben, bei dem eine Vielzahl von Akupunkturnadeln an einem Haltering befestigt in einem hülsenförmigen Behälter angeordnet sind. Dieser Behälter erlaubt zwar die Aufnahme einer Vielzahl von Akupunkturnadeln und im Verbund mit der Handakupunkturvorrichtung das Setzen der Nadeln ohne eine manuelle Berührung. Ein wesentlicher Nachteil besteht jedoch darin, dass die Akupunkturnadeln in dem herkömmlichen Behälter nicht unter sterilen Bedingungen geschützt aufbewahrt werden können. Außerdem ist der herkömmliche Behälter für eine Bereitstellung einzelner Akupunkturnadeln für ein manuelles Setzen ungeeignet.

Aus US 2001/0014792 ist ein Nadelmagazin für Spritzennadeln bekannt, die unter sterilen Bedingungen in Kompartimenten eines Magazinkörpers angeordnet sind. Zur Anwendung wird jeweils eine Nadel mit einem Spritzenkolben aus dem Magazin entnommen. Das herkömmliche Nadelmagazin besitzt einen komplizierten und zur Anwendung mit speziell angepassten Spritzen beschränkten Aufbau. Das Nadelmagazin ist zur Bereitstellung abweichend geformter Nadeln oder Werkzeuge ungeeignet.

Die oben beschriebenen Nachteile sind nicht nur bei Behältern oder Dispensern für Spritzen- oder Akupunkturnadeln gegeben. Sie treten vielmehr auch bei allen anderen Anwendungen auf, bei denen Sterilgut zuverlässig geschützt gelagert und mit geringem Bedienaufwand bereitgestellt werden soll.

Die Aufgabe der Erfindung ist es, einen verbesserten Behälter zur Aufnahme von Sterilgut vorzuschlagen, mit dem die Nachteile der herkömmlichen Behälter überwunden werden und der einen erweiterten Anwendungsbereich besitzt. Der Behälter soll insbesondere einen verbesserten Schutz gegen Umgebungseinflüsse, einen vereinfachten Aufbau und eine vereinfachte Bedienbarkeit aufweisen. Der Behälter soll insbesondere für eine dichte Packung einer Vielzahl von steril zu lagernden Teilen und eine Automatisierung der Beschickung ermöglichen. Eine weitere Aufgabe der Erfindung ist es, einen verbesserten Dispenser für Sterilgut, wie z.B. Spritzen- oder Akupunkturnadeln bereitzustellen, mit dem die Nachteile herkömmlicher. Dispenser oder Magazine überwunden werden. Der Dispenser soll sich insbesondere durch eine vereinfachte Bedienbarkeit und hohe Flexibilität bei der Anpassung an verschiedene Aufgaben besitzen. Eine weitere Aufgabe der Erfindung besteht darin, ein verbessertes Verfahren zur Bereitstellung von Sterilgut, wie z.B. von Akupunktur- oder Spritzennadeln vorzuschlagen, mit dem die Nachteile herkömmlicher Anwendungen von Sterilgut-Behältern oder -Dispensern überwunden werden.

Diese Aufgaben werden durch einen Behälter zur Aufnahme von Sterilgut, einen Dispenser für Sterilgut und ein Verfahren zur Bereitstellung von Sterilgut mit den Merkmalen gemäß den Patentansprüchen 1, 25 oder 35 gelöst. Vorteilhafte Ausführungsformen und Anwendungen der Erfindung ergeben sich aus den abhängigen Ansprüchen.

Vorrichtungsbezogen wird die Aufgabe der Erfindung gemäß einem ersten Gesichtspunkt durch einen Behälter zur Aufnahme von Sterilgut mit einem Behälterkörper gelöst, durch den sich mindestens ein Aufnahmekanal erstreckt, der zumindest an einem Ende, wo sich der Aufnahmekanal zur Oberfläche des Behälterkörpers öffnet, eine Abdeckung aufweist, die eine sterile Barriere gegen das Eindringen unerwünschter Verunreinigungen von der Umgebung in den Aufnahmekanal bildet, wobei im Aufnahmekanal zwischen der Abdeckung und einem verschiebbaren Ausstoßstempel ein Hohlraum zur Aufnahme des Sterilguts gebildet ist.

Die erfindungsgemäße Verwendung eines kompakten Behälterkörpers mit einem Aufnahmekanal, aus dem das jeweilige Sterilgut, z. B. eine Spritzen- oder Akupunkturnadel durch Verschiebung des Ausstoßstempels freigegeben werden kann, besitzt die folgenden Vorteile. Erstens ist der Behälterkörper wiederholt sterilisierbar und wiederverwendbar; so dass der Verpackungsabfall vermindert wird. Des Weiteren erfolgt die sterile Einbettung im Behälter mit erhöhter Sicherheit. Das Sterilgut wird im Aufnahmekanal im Wesentlichen von dessen sterilen Innenwänden umgeben, von denen keine Verunreinigungen auf das Sterilgut übergehen. Nach außen ist das Sterilgut an der Öffnung des Aufnahmekanals durch die Abdeckung abgeschirmt, die beispielsweise im Vergleich zu Blisterverpackungen eine verminderte Fläche besitzt, so dass das Risiko einer unbeabsichtigten Verletzung der Abdeckung verringert ist. Die Abdeckung, die zur Freigabe des Sterilguts zerstörbar ist, kann bei ausreichender Stabilität mit verminderter Dicke gebildet sein. Ein weiterer wichtiger Vorteil besteht darin, dass das Sterilgut einhändig freigegeben werden kann, so dass für den Nutzer die zweite Hand zur Übernahme des freigegebenen Sterilguts frei bleibt. Schließlich besitzt der erfindungsgemäße Behälter eine universale Anwendbarkeit für die verschiedensten Typen von steril zu lagernden Gegenständen (insbesondere Vorrichtungen, Vorrichtungsteile oder Materialien). Im Aufnahmekanal, der vorzugsweise eine langgestreckte, gerade Form besitzt, können die verschiedensten Teile, wie z. B. Einwegpipetten, Zucker-Testlanzetten, Skalpellmesser, Kanülennadeln, Akupunkturnadeln, Implantatmaterialien und dergleichen geschützt positioniert und durch Verschiebung des Ausstoßstempels freigegeben werden.

Wenn gemäß einer bevorzugten Ausführungsform der Erfindung der Ausstoßstempel mit einem weiteren Hohlraum, wie z. B. mit einer inneren Bohrung ausgestattet ist, können sich Vorteile für die stabile Positionierung des Sterilguts im Aufnahmekanal und die Verschiebung des Sterilguts mit dem Ausstoßstempel ergeben. In die innere Bohrung kann ein Teil des Sterilguts, wie z. B. das Nadelteil einer Akupunkturnadel oder eines Kanülenaufsatzes einer Spritze oder eine Schneide eines chirurgischen Werkzeugs hineinragen, während sich das übrige Sterilgut, wie z.B. der Nadelgriff einer Akupunkturnadel oder der Schaft des chirurgischen Werkzeugs im Hohlraum zwischen dem Ausstoßstempel und der Abdeckung des Aufnahmekanals befindet. Vorteilhafterweise wird eine doppelte Führung des Sterilguts einerseits im Aufnahmekanal und andererseits in der inneren Bohrung des Ausstoßstempels realisiert, so dass bei der Freigabe des Sterilguts aus dem erfindungsgemäßen Behälter das Sterilgut bis zur endgültigen manuellen Entnahme oder Entfernung mittels eines Hilfsgerätes ausreichend stabil am Behälter gehaltert wird.

Gemäß einer weiteren bevorzugten Ausführungsform der Erfindung ist der Ausstoßstempel im Aufnahmekanal formschlüssig angeordnet. Vorteilhafterweise kann damit die Gefahr vermindert werden, dass von der Seite des Ausstoßstempels her unerwünscht Verunreinigungen zum Sterilgut gelangen. Ein zusätzlicher Schutz wird erreicht, wenn gemäß einer weiteren Variante der Erfindung auch das rückseitige Ende des Aufnahmekanals mit einer Abdeckung ausgestattet ist.

Die Abdeckung an der Stirn- und ggf. an der Rückseite des Behälters besteht erfindungsgemäß aus einem schichtförmigen Material, das vorzugsweise manuell durch Vorschieben des Ausstoßstempels mit dem Sterilgut zerstörbar ist. In diesem Fall kann auf ein Zusatzwerkzeug zur Durchbrechung der sterilen Abdeckung verzichtet werden. Besonders vorteilhaft in Bezug auf die Verfügbarkeit von Materialien zur Bildung einer Sterilitätsbarriere ist die Verwendung von an sich verfügbaren Laminaten auf der Grundlage von Papier und/oder Kunststofffolie, wie z. B. von so genanntem "Medical Paper".

Der mindestens eine Aufnahmekanal im Behälterkörper kann in Abhängigkeit von der konkreten Anwendung mit den verschiedensten Querschnittsformen gebildet sein. Es kann beispielsweise ein rechteckiger, elliptischer oder kreisrunder Querschnitt vorgesehen sein. Für eine hohe Stabilität der Lagerung und eine klemmfreie Verschiebung des Ausstoßstempels wird jedoch, insbesondere für stab- oder zylinderförmiges Sterilgut ein kreisrunder Querschnitt bevorzugt. Ein zylinderförmiger Aufnahmekanal besitzt den zusätzlichen Vorteil, dass die Fläche der Innenwand für ein vorgegebenes Volumen minimiert ist und dadurch die Sterilisierung vereinfacht und das Verunreinigungsrisiko vermindert werden. Besonders bevorzugt wird der mindestens eine Aufnahmekanal mit einem Kanalinnendurchmesser gebildet, der wenigstens 1 mm beträgt.

Eine besonders bevorzugte Anwendung des erfindungsgemäßen Behälters besteht in der Aufnahme von mindestens einer Akupunkturnadel mit einem Nadelgriff und einem Nadelteil, wobei vorzugsweise ein Aufnahmekanal mit kreisförmigem Querschnitt und einem Innendurchmesser vorgesehen ist, der größer oder gleich dem Außendurchmesser des Nadelgriffes ist.

Für die Aufnahme von Akupunkturnadeln ist es besonders vorteilhaft, wenn die innere Bohrung des Ausstoßstempels zur Aufnahme des Nadelteils eingerichtet ist. Neben der obengenannten doppelten Führung wird damit vorteilhafterweise ein besonderer Schutz für die Nadelspitze bereitgestellt, die generell ein wichtiges Qualitätsmerkmal der Akupunkturnadel ist. Für eine erhöhte Stabilität der Positionierung der Akupunkturnadel im Aufnahmekanal kann es von Vorteil sein, wenn der Durchmesser der inneren Bohrung des Ausstoßstempels kleiner als der Außendurchmesser des Nadelgriffes ist. Damit wird die Gefahr vermieden, dass die Akupunkturnadel komplett in den Ausstoßstempel rutscht oder bei dessen Verschiebung in der inneren Bohrung des Ausstoßstempels festklemmt. Der Nadelgriff bildet eine Auflage, an der bei der Verschiebung des Ausstoßstempels die Kraftübertragung erfolgt.

Vorteilhafterweise kann die Erfindung auch mit so genannten Führungsröhrchen-Akupunkturnadeln angewendet werden. Bei diesen bildet das Sterilgut eine Kombination aus einem schützenden Führungsröhrchen und einer Nadel mit einem Nadelgriff und einer Nadelspitze, wobei die Nadel z. B. mit einer Klebeverbindung im Inneren des Führungsröhrchens fixiert ist. Bei dieser Variante wird der Ausstoßstempel des erfindungsgemäßen Behälters durch das Führungsröhrchen gebildet. Die innere Bohrung des Ausstoßstempels ist entsprechend geringfügig größer als der Nadelgriff gewählt. Die Verwendung der Führungsröhrchen-Akupunkturnadeln besitzt den Vorteil, dass Sterilitätsprobleme bei der Beschickung des Behälters vermieden werden können.

Da bei der Verwendung mit Führungsröhrchen-Aküpunkturnadeln über den Nadelgriff nicht genügend Kraft übertragen werden kann, um die Abdeckung zu durchbrechen, ist gemäß einer weiteren Ausführungsform der Erfindung der Behälter mit einem Dorn ausgestattet, auf den direkt vom Ausstoßstempel, d.h. vom Führungsröhrchen eine Kraft zum Öffnen der Abdeckung übertragen werden kann.

Wenn der Dorn an seinem vorderen Ende mit einer Schnittfläche ausgestattet ist, kann das Öffnen der Abdeckung vom Inneren des Aufnahmekanals her erheblich erleichtert werden. Weitere Vorteile für die Handhabbarkeit des erfindungsgemäßen Behälters ergeben sich, wenn der Dorn ein hohles Bauteil mit einem innen durchgehenden Kanal bildet, durch den das Sterilgut bei geöffneter Abdeckung nach außen geschoben werden kann. In diesem Fall wird der Dorn nur zum Öffnen der Abdeckung benötigt. Das Sterilgut kann aus dem Behälter entnommen werden, ohne dass der Dorn vom Behälter getrennt werden muss.

Um das Zurückhalten des Dorns am Behälter zu verbessern, ist gemäß einer weiteren Modifikation der Erfindung der Dorn mit einem radial nach außen vorragenden, z. B. umlaufenden oder seitlich abstehenden Vorsprung ausgestattet. Der Vorsprung ist auf der Außenseite des Dorns vorzugsweise mit einem Abstand vom vorderen Ende des Dorns und insbesondere von der Schnittfläche angeordnet. Der Abstand ist so gewählt, dass sich der Vorsprung im vorgeschobenen Zustand des Dorns nach Öffnung der Abdeckung noch im Aufnahmekanal unter der geöffneten Abdeckung befindet. Vorteilhafterweise wird damit erreicht, dass, wenn der Dorn zum Öffnen der Abdeckung aus dem Aufnahmekanal geschoben wird, der Vorsprung und damit der Dorn unter der geöffneten Abdeckung zurückgehalten wird oder in einen Spalt zwischen dem Behälterkörper und der Abdeckung greifen kann, so dass eine weitere Verschiebung des Dorns vermieden wird.

Auf seinem rückseitigen, zum Sterilgut weisenden Ende ist der Dorn gemäß einer bevorzugten Ausführungsform der Erfindung mit einer Hülse ausgestattet, die zumindest während des Öffnens der Abdeckung eine Auflage für den Ausstoßstempel bildet. Der Dorn oder wenigstens die Hülse besitzt vorzugsweise einen veränderlichen Innendurchmesser, so dass beim Öffnen der Abdeckung in einer ersten Phase durch die Hülse der Anschlag gebildet wird, während nach dem Öffnen der Abdeckung die Hülse sich erweitert und das Sterilgut komplett durch lässt. Hierzu besteht die Hülse vorzugsweise aus einem elastisch federnden, im Aufnahmekanal des Behälterkörpers vorgespannten Material. Bei dieser Ausführungsform der Erfindung weist der Aufnahmekanal an seinem Ende, das mit der Abdeckung geschlossen ist, eine Dornaufnahme mit einem erweiterten Durchmesser auf. In der Dornaufnahme ist der Durchmesser des Aufnahmekanals größer als im übrigen Kanal. Die Dimensionierung der Dornaufnahme ermöglicht die Entspannung der Hülse des vorgeschobenen Dorns, sobald dieser die Abdeckung geöffnet hat.

Besondere Vorteile für eine möglichst vielseitige Anwendung des erfindungsgemäßen Behälters für verschiedene Typen von Sterilgut, z. B. für verschiedene Typen von Akupunkturnadeln mit verschiedenen Griff- und Nadelgrößen werden realisiert, wenn die Stempellänge des Ausstoßstempels so gewählt wird, dass die Summe aus der Stempellänge und der maximalen Länge von in der Praxis interessierenden Nadelgriffen von Akupunkturnadeln gleich oder geringfügig kleiner als die Kanallänge des Aufnahmekanals ist. In diesem Fall kann mit einem einheitlichen Ausstoßstempel der erfindungsgemäße Behälter passend für alle in der Praxis interessierenden Typen von Akupunkturnadeln verwendet werden.

Die Verschiebung des Ausstoßstempels zur Freigabe des Sterilguts erfolgt vorzugsweise durch Ausübung einer Vortriebskraft von der Rückseite des Behälterkörpers durch den Aufnahmekanal. Hierzu kann der erfindungsgemäße Behälter gemäß einer weiteren Variante mit einem Betätigungsteil zur Verschiebung des Ausstoßstempels ausgestattet sein. Gemäß einer bevorzugten Ausgestaltung weist das Betätigungsteil einen Betätigungsstift auf, dessen Größe an den Innendurchmesser des Aufnahmekanals angepasst ist.

Gemäß einer weiteren besonders bevorzugten Ausführungsform der Erfindung weist der Behälterkörper eine Vielzahl von Aufnahmekanälen auf. Im Unterschied zu den oben genannten Blisterverpackungen können die Aufnahmekanäle und damit das Sterilgut mit einer hohen Dichte im erfindungsgemäßen Behälter angeordnet werden. Des Weiteren wird ein Reservoir mit vielen einzelnen Sterilgut-Teilen, z. B. Akupunkturnadeln bereitgestellt, das ein dauerhaftes und gleichförmiges Arbeiten mit dem Sterilgut ermöglicht.

Wenn die Aufnahmekanäle von einer Rückseite des Behälterkörpers zu dessen Stirnseite parallel verlaufen, können sich Vorteile für eine hohe räumliche Lagerdichte des Sterilguts ergeben.

Allgemein kann der Behälterkörper mit jeder Volumenform gebildet sein, die an die jeweilige Anwendung des erfindungsgemäßen Behälters angepasst ist. Gemäß einer bevorzugten Variante besitzt der Behälterkörper die Form eines geraden Kreiszylinders. In diesem Fall können sich Vorteile für einen besonders kompakten Aufbau, eine vereinfachte Ausrichtung des Behälters in einem erfindungsgemäßen Dispenser (s. u.) und eine vereinfachte, automatisierbare Verpackung des Sterilguts im erfindungsgemäßen Behälter, z. B. auf einem Rundtakttisch ergeben. Für eine reproduzierbare Einstellung des Behälterkörpers relativ zu einer Betätigungseinrichtung kann es von Vorteil sein, wenn die Aufnahmekanäle im Behälterkörper so angeordnet sind, dass in einer gegebenen Querschnittsfläche des Behälterkörpers die Schnittpunkte der durchgehenden Aufnahmekanäle ein vorbestimmtes Muster ergeben. Bei der Verwendung eines zylinderförmigen Behälterkörpers liegen die genannten Schnittpunkte vorzugsweise auf einer Vielzahl koaxialer Kreise. Dabei sind die Aufnahmekanäle vorzugsweise radial fluchtend angeordnet. Dies bedeutet, dass die genannten Schnittpunkte radial benachbarter Aufnahmekanäle mit der betrachteten Querschnittsfläche auf Geraden liegen, welche die Zylinderachse schneiden.

Gemäß einer alternativen Variante besitzt der Behälterkörper die Form eines Quaders, in dem die Aufnahmekanäle reihen- oder matrixförmig angeordnet sind.

Ein weiterer Vorteil der Erfindung ist gegeben, wenn die Abdeckung der Aufnahmekanäle auf den Stirnseiten und ggf. den Rückseiten des Behälterkörpers jeweils aus einem einstückigen, schichtförmigen Material gebildet ist. Damit kann bei der Verpackung von Sterilgut die Bildung der sterilen Barriere durch Aufbringen der Abdeckung vereinfacht werden.

Vorrichtungsbezogen wird die obengenannte Aufgabe gemäß einem weiteren Gesichtspunkt der Erfindung durch die Bereitstellung eines Dispensers für Sterilgut, insbesondere für Akupunktur- oder Spritzennadeln gelöst, der eine Betätigungseinrichtung aufweist, an der ein erfindungsgemäßer Behälter angebracht ist und mit welcher der Ausstoßstempel in dem mindestens einen Aufnahmekanal betätigbar ist. Der erfindungsgemäße Dispenser bildet vorteilhafterweise ein kompaktes Gerät mit einem breiten Anwendungsbereich für die verschiedensten Typen von keimfrei zu lagerndem Sterilgut. Der erfindungsgemäße Dispenser kann vorteilhafterweise einhändig bedient werden.

Gemäß einer bevorzugten Ausführungsform der Erfindung ist die Betätigungseinrichtung mit einem Betätigungsstift zur Verschiebung des mindestens einen Ausstoßstempels in einem Gehäuse untergebracht, das einen Handgriff oder eine Wandhalterung des erfindungsgemäßen Dispensers bildet. Gemäß einer bevorzugten Variante ist der Betätigungsstift mit einer manuell betätigbaren Antriebseinrichtung beweglich. Alternativ kann eine elektrisch betriebene Antriebseinrichtung vorgesehen sein. Besondere Vorteile für eine manuelle Betätigung z. B. mit einem einzelnen Finger können sich ergeben, wenn die Antriebseinrichtung eine Vorschubstange im Gehäuse umfasst, an welcher der Betätigungsstift befestigt ist. Wenn der Betätigungsstift exzentrisch verstellbar angeordnet ist, können vorteilhafterweise verschiedene radiale Betriebspositionen eingestellt werden. Dies ermöglicht die Verwendung eines einzigen Betätigungsstiftes auch bei Behältern mit einer Vielzahl von Aufnahmekanälen, die beispielsweise koaxial in einem zylinderförmigen Behälterkörper angeordnet sind.

Zusätzliche Vorteile für einen vereinfachten Betrieb des erfindungsgemäßen Dispensers können sich ergeben, wenn die Antriebseinrichtung zusätzlich mit einer Einstelleinrichtung zur Ausrichtung des erfindungsgemäßen Behälters relativ zum Betätigungsstift ausgestattet ist. Beispielsweise kann der zylinderförmige Behälterkörper drehbar auf der Vorschubstange angeordnet und jeweils passend zum Betätigungsstift ausgerichtet werden. Diese Ausrichtung wird vorteilhafterweise noch vereinfacht, wenn eine Rasteinrichtung vorgesehen ist, die den Behälterkörper auf bestimmte Positionen einstellt, die gerade den Positionen der Aufnahmekanäle im Behälterkörper entsprechen. Damit wird eine systematische Freigabe des Sterilguts z. B. entsprechend der Reihenfolge der Aufnahmekanäle erleichtert.

Wenn das Gehäuse der Betätigungseinrichtung als Handgriff gebildet ist, der sich parallel zu den Aufnahmekanälen im Behälterkörper erstreckt, können sich Vorteile für die einhändige Bedienung des Dispensers ergeben. Alternativ kann eine Ausrichtung senkrecht zu den Aufnahmekanälen vorgesehen sein, was für bestimmte Prozeduren, bei denen beispielsweise mehrere Personen auf den Dispenser zugreifen, von Vorteil sein kann.

Verfahrensbezogen wird die obengenannte Aufgabe der Erfindung durch die Bereitstellung von Sterilgut, insbesondere von Akupunktur- oder Spritzennadeln mit dem erfindungsgemäßen Dispenser oder dem erfindungsgemäßen Behälter dahingehend gelöst, dass jeweils das Sterilgut durch eine Verschiebung des Ausstoßstempels im Aufnahmekanal verschoben wird, bis durch das Sterilgut die Abdeckung durchbrochen wird und der freiliegende Teil des Sterilguts zur weiteren Verwendung aus dem Behälterkörper herausragt.

Vorzugsweise wird bei der Anwendung mit Akupunkturnadeln das Sterilgut so aus dem Aufnahmekanal verschoben, dass zunächst der Nadelgriff freigegeben wird, der dann beispielsweise von einem Arzt erfasst und in die Haut einer zu behandelnden Person gesetzt werden kann.

Weitere Einzelheiten und Vorteile der Erfindung werden aus der folgenden Beschreibung der beigefügten Zeichnungen ersichtlich. Es zeigen:
- Figuren 1 und 2:: eine schematische Schnittdarstellung einer ersten Ausführungsform eines erfindungsge- mäßen Sterilgut-Behälters mit einem ein- zelnen Aufnahmekanal;
- Figur 3:: Illustrationen eines erfindungsgemäß ver- wendeten Ausstoßstempels im Verbund mit einer Akupunkturnadel;
- Figur 4:: eine schematische Schnittdarstellung einer weiteren Ausführungsform eines erfindungs- gemäßen Sterilgut-Behälters, der mit einem Dorn ausgestattet ist;
- Figur 5:: Illustrationen verschiedener Phasen des Ausstoßens von Sterilgut aus einem erfin- dungsgemäßen Sterilgut-Behälter gemäß der in Fig. 4 gezeigten Ausführungsform;
- Figur 6:: Illustrationen von Einzelheiten einer wei- teren Ausführungsform eines erfindungsge- mäßen Sterilgut-Behälters, der mit einem Dorn ausgestattet ist;
- Figur 7:: eine schematische Illustration einer wei- teren Ausführungsform des erfindungsgemä- ßen Sterilgut-Behälters mit einer Vielzahl von Aufnahmekanälen;
- Figuren 8 bis 11:: Illustrationen einer weiteren Ausführungs- form eines erfindungsgemäßen Sterilgut- Behälters mit einer Vielzahl von Aufnahme- kanälen; und
- Figuren 12 und 13:: verschiedene Ausführungsformen erfindungs- gemäßer Sterilgut-Dispenser.

Die Erfindung wird im Folgenden beispielhaft unter Bezug auf Behälter und Dispenser für Akupunkturnadeln beschrieben. Es wird betont, dass die Umsetzung der Erfindung nicht auf diese Anwendung beschränkt, sondern analog mit anderen in der Medizin, Biologie und Biochemie interessierenden Typen von Sterilgut, z. B. Spritzennadeln, Medikamentkapseln möglich ist.

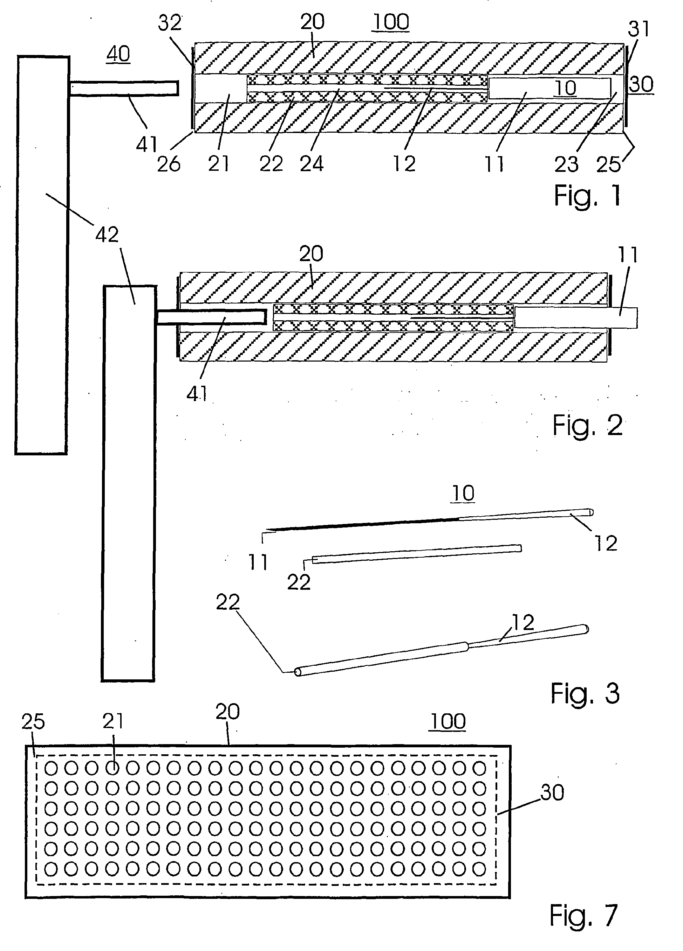

Die Figuren 1 und 2 zeigen in schematischer Schnittansicht eine erste Ausführungsform eines erfindungsgemäßen Behälters 100 im geschlossenen (Figur 1) und im geöffneten (Figur 2) Zustand. Der Behälter 100 zur Aufnahme der Akupunkturnadel 10 umfasst einen Behälterkörper 20 mit einem Aufnahmekanal 21 und einem Ausstoßstempel 22 und eine Abdeckung 30.

Der Behälterkörper 20 besteht aus einem Kunststoff, z. B. Polyethylen. Er ist beispielsweise durch ein Spritzgussverfahren oder als Strangpressprofil hergestellt. Die äußere Form des Behälterkörpers 20 ist in Abhängigkeit von den Anforderungen der konkreten Anwendung gewählt. Es kann beispielsweise eine Zylinder- oder Quaderform vorgesehen sein. Die Oberfläche des Behälterkörpers 20 kann vorteilhafterweise als Informationsträger dienen. Der Aufnahmekanal 21 verläuft als gerader Kanal von der Stirnseite 25 zur Rückseite 26 des Behälterkörpers 20. An den Enden öffnet sich der Aufnahmekanal in die Oberfläche des Behälterkörpers 20. Im Aufnahmekanal 21 ist der Ausstoßstempel 22 verschiebbar angeordnet.

Der Ausstoßstempel 22 besitzt die Form einer Hülse oder eines Röhrchens mit einer inneren Bohrung 24. Der Außendurchmesser des Ausstoßstempels 22 ist gleich dem Innendurchmesser des Aufnahmekanals 21. Vorteilhafterweise wird dadurch die Verschiebbarkeit des Ausstoßstempels 22 gewährleistet, ohne dass dieser durch Trägheitsbewegungen im Aufnahmekanal 21 verrutscht. Wenn ein derartiges Verrutschen allerdings unkritisch ist, kann der Außendurchmesser des Ausstoßstempels geringer als der Innendurchmesser des Aufnahmekanals 21 sein. Der Ausstoßstempel besteht vorzugsweise aus dem selben Material wie der Behälterkörper 20.

Die Stempellänge (axiale Länge) des Ausstoßstempels 22 ist geringer als die Kanallänge des Aufnahmekanals 21. Dadurch wird auf der Stirnseite des Behälters 100 ein Hohlraum 23 zur Aufnahme des Sterilguts (hier: der Akupunkturnadel) gebildet. Je nach der verwendeten Nadelgröße kann im Aufnahmekanal auch auf der Rückseite noch ein Hohlraum verbleiben. Das Sterilgut 10 ist im illustrierten Fall eine Akupunkturnadel mit einem Nadelgriff 11 und einem Nadelteil 12. Das Nadelteil 12 ragt in die innere Bohrung 24 des Ausstoßstempels 22 hinein.

Die Abdeckung 30 besteht jeweils aus Papier- oder Folienabdeckungen (31, 32) auf den Stirn- und Rückseiten 25, 26 des Behälterkörpers 20. Es wird beispielsweise Medical Paper verwendet. Das Papier oder die Folie 31, 32 ist auf dem Behälterkörper 20 in an sich bekannter Weise aufgeklebt.

Der Behälterkörper 20 und der Aufnahmekanal 21 sind so dimensioniert, dass die Oberfläche, welche die Austrittsöffnung des Aufnahmekanals 21 an den Stirn- und Rückseiten 25, 26 umgibt, genügend groß ist, um im Verbund mit der Abdeckung einer ausreichende Sperrwirkung zu erzielen.

Die Figuren 1 und 2 zeigen auch ein Betätigungsteil 40, das zur Freigabe der Akupunkturnadel 10 aus dem Behälter 100 eingerichtet ist. Das Betätigungsteil 40 umfasst einen Betätigungsstift 41 und einen Griff 42. Die Länge des Betätigungsstiftes 41 wird in Abhängigkeit von den Dimensionen des Behälters, insbesondere des Aufnahmekanals 21 und des Ausstoßstempels 22 derart gewählt, dass die Akupunkturnadel 10 sicher aus dem Aufnahmekanal 21 ausgestoßen werden kann.

Figur 1 zeigt den erfindungsgemäßen Behälter 100 im geschlossenen Zustand. Die Akupunkturnadel 10 befindet sich in einer sterilen Umgebung im Hohlraum 23 des Aufnahmekanals 21. Zur Freigabe der Akupunkturnadel 10 wird der Betätigungsstift 41 in die Rückseite 26 des Behälterkörpers 20 eingeführt. Mit dem Betätigungsstift 41 wird der Ausstoßstempel 22 verschoben, bis der Nadelgriff 11 die Abdeckfolie 31 auf der Stirnseite 25 durchbricht. In diesem Zustand (Figur 2) ragt der Nadelgriff 11 aus dem Behälterkörper 20 vor, so dass die manuelle Entnahme und Anwendung realisiert werden kann.

Die Länge des Behälterkörpers 20 beträgt beispielsweise 70 bis 80 mm. Der Durchmesser des Aufnahmekanals 21 beträgt beispielsweise 2.5 mm. Die Länge des Ausstoßstempels 22 ist so gewählt, dass im Aufnahmekanal 21 der Hohlraum 23 zur Aufnahme der üblichen Typen von Akupunkturnadeln 10 ausreichend groß ist. Sie beträgt beispielsweise 50 mm. Die Sperrfläche um die Austrittsöffnungen des Aufnahmekanals besitzt beispielsweise eine radiale Breite von 7 mm. Auf der Rückseite 26 kann der Behälterkörper 20 z. B. durch einen Faltenbalg und/oder die Abdeckung als Folie elastisch verformbar gebildet sein, so dass auch bei Betrieb des Betätigungsteil 40 der Kanal 21 rückseitig verschlossen bleibt.

Figur 3 illustriert die Akupunkturnadel 10 und den Ausstoßstempel 22 als getrennte Teile (oben) und im Verbund (unten). Vorteilhafterweise ermöglicht die Erfindung eine doppelte Führung der Akupunkturnadel 10 dahingehend, dass einerseits das Nadelteil 12 im Ausstoßstempel 22 und andererseits der Ausstoßstempel 22 im Aufnahmekanal 21 geführt wird.

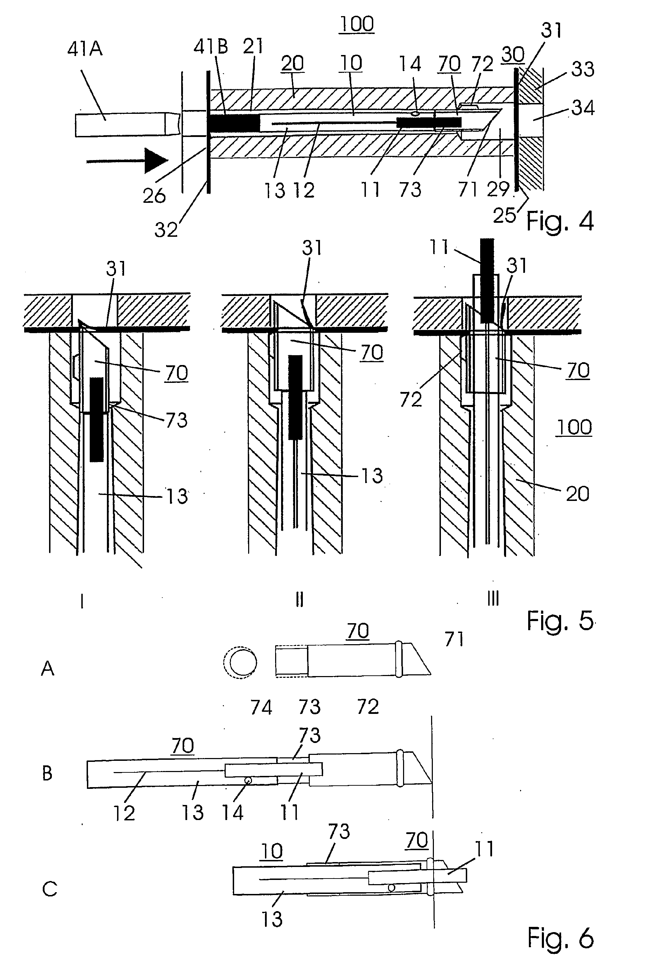

In Figur 4 ist eine zweite Ausführungsform eines erfindungsgemäßen Behälters 100 im geschlossenen Zustand illustriert. Diese Ausführungsform der Erfindung ist für Führungsröhrchen-Akupunkturnadeln 10 angepasst, die einen Nadelgriff 11, ein Nadelteil 12 und ein Führungsröhrchen 13 umfassen. Der Nadelgriff 11 ist mittels einer punktförmigen Klebeverbindung 14 (s. auch Figur 6) auf der inneren Oberfläche des Führungsröhrchens 13 fixiert.

Der Aufnahmekanal 21 im Behälterkörper 20 verläuft wie bei der Ausführungsform gemäß Fig. 1 als gerader Kanal zwischen den Stirn- und Rückseiten 25, 26, wobei in diesem Fall jedoch am stirnseitigen Ende des Aufnahmekanals 21 eine Dornaufnahme 29 vorgesehen ist. Der Innendurchmesser der Dornaufnahme 29 ist größer als der Innendurchmesser des übrigen Aufnahmekanals 21.

Der Aufnahmekanal 21 ist auf den Oberflächen des Behälterkörpers 20 mit einer Abdeckung 30 verschlossen, die wie bei Fig. 1 Papier- oder Folienabdeckungen 31, 32, z. B. aus Medical Paper umfasst. Figur 4 zeigt als weiteres Teil der Abdeckung 30 eine so genannte Druckscheibe 33 mit einer durchgehenden Öffnung 34 entsprechend der Lage des Aufnahmekanals 21 bzw. der Dornaufnahme 29. Mit der Druckscheibe 33 kann ein unbeabsichtigtes Abziehen der Papier- oder Folienabdeckung 31 verhindert werden. Die Öffnung 34 stellt eine zusätzliche Führung für den Dorn 70 und/oder das Sterilgut dar.

Bei der in Fig. 4 gezeigten Ausführungsform der Erfindung ist des Weiteren ein hohler Dorn 70 vorgesehen, der eine Schnittfläche 71, einen radialen Vorsprung 72 und eine elastische Hülse 73 umfasst, bei welcher der Innendurchmesser des Dorns veränderlich ist. Der Dorn 70 sitzt vor dem Ausstoßen der Akupunkturnadel 10 so im Aufnahmekanal 21, dass die Schnittfläche 71 zur Abdeckung 31 weisend und die Hülse 73 in einem vorgespannten Zustand im Aufnahmekanal 21 angeordnet sind. Der seitliche Vorsprung 72 ist in der Dornaufnahme 29 des Aufnahmekanals 21 angeordnet.

Bei der in Fig. 4 gezeigten Ausführungsform der Erfindung umfasst das Betätigungsteil 40 zwei getrennte Betätigungsstifte 41A, 41B. Der erste Betätigungsstift 41A ist wie in Fig. 1 mit dem Griff 42 oder einer anderweitigen mechanischen Komponente des Betätigungsteils 40 verbunden, während der zweite Betätigungsstift 41B im Aufnahmekanal 21 angeordnet ist. Bei dieser Gestaltung übernimmt das Führungsröhrchen 13 der Akupunkturnadel 10 die Funktion des in Fig. 1 gezeigten Ausstoßstempels 22.

Wenn zum Ausstoßen der Akupunkturnadel 10 das Betätigungsteil 40 betätigt wird, so dass die Betätigungsstifte 41A, 41B in Pfeilrichtung vorwärts bewegt werden, ergeben sich die in Fig. 5 illustrierten Phasen der Freigabe der Akupunkturnadel 10. In einer ersten Phase bildet die Hülse 73 einen Anschlag für das Führungsröhrchen 13, so dass der Dorn 70 zur Abdeckung 31 bewegt wird. Mit der Schnittfläche 71 wird die Abdeckung 31 durchstoßen (Phase I).

Bei der weiteren Bewegung kann sich die Hülse 73 oder wie gezeigt der gesamte Dorn 70 in der Dornaufnahme 29 entspannen (Phase II), so dass die Hülse 73 oder der gesamte Dorn 70 sich weitet und nicht länger einen Anschlag für das Führungsröhrchen 13 bildet. Beim weiteren Vorschub kann das Führungsröhrchen 13 mit dem vorragenden Nadelgriff 11 durch den hohlen Dorn 70 vorgeschoben werden (Phase III), während der Dorn 70 mit dem Vorsprung 72 durch Reste der Abdeckung 31 zurückgehalten wird. Im weiteren Verfahren kann der Nadelgriff 11 manuell erfasst und aus dem Behälter 100 herausgezogen werden. Durch ein geringfügiges Verkanten des Nadelgriffs 11 kann die Klebeverbindung unterbrochen werden, so dass das Führungsröhrchen 13 im Behälterkörper 20 zurückbleibt.

Figur 6 illustriert Einzelheiten der zweiten Ausführungsform der Erfindung, bei der wie in Fig. 4 ein Dorn 70 im Behälterkörper vorgesehen ist. In Teilbild A von Fig. 6 ist eine Seitenansicht des Dornes 70 mit der Schnittfläche 71, dem bei dieser Ausführungsform umlaufenden Vorsprung 72 und der Hülse 73 gezeigt. Die Hülse 73 umfasst ein federndes Bauteil in Form eines Zylinders, in dessen Mantel eine longitudinal (axial) verlaufende Unterbrechung (Lücke oder Überlappung) vorgesehen ist. Sie ist in einem kurzen Abschnitt (z. B. bei 74) mit dem übrigen Körper des Dornes 70 einstückig verbunden ist und im übrigen einen variablen Durchmesser aufweist. Der Durchmesser kann durch eine Verbiegung des Mantels der Hülse verändert werden. Der Durchmesser kann sich insbesondere von einem vorgespannten, teilweise aufgerollten Zustand im Aufnahmekanal 21 zu einem entspannten Zustand (gestrichelt eingezeichnet) in der Dornaufnahme 29 vergrößern. In Teilbild B ist illustriert, wie die Hülse 73 im gespannten Zustand einen Anschlag für das Führungsröhrchen 13 der Akupunkturnadel 10 bildet. Vorteilhafterweise wird in diesem Zustand auf den Nadelgriff 11, der über die Klebeverbindung 14 mit dem Führungsröhrchen 13 verbunden ist, und das Nadelteil 12 keine Kraft ausgeübt.

Teilbild C zeigt analog zu Fig. 5 die Phase III der Ausstoßbewegung der Akupunkturnadel 10. In diesem Zustand ragt das Führungsröhrchen 13 durch die entspannte Hülse 73 hindurch, bis der Nadelgriff 11 vom Dorn 70 hervorragt und manuell entnommen werden kann.

Figur 7 zeigt beispielhaft eine Ausführungsform des erfindungsgemäßen Behälters 100 mit einem Behälterkörper 20, in dem eine Vielzahl von Aufnahmekanälen 21 gebildet sind. Es ist die Draufsicht auf die Stirnseite 25 eines quaderförmigen Behälterkörpers 20 dargestellt. Die gestrichelte Linie zeigt die Ausdehnung des als Abdeckung 30 verwendeten Medical Paper. Die Aufnahmekanäle 21 sind gerade und parallel zu einer Seitenfläche des Behälterkörpers 20 verlaufend so angeordnet, dass ihre Austrittsöffnungen (oder Schnittpunkte mit einer Querschnittsfläche des Behälterkörpers 20) eine regelmäßige Anordnung z. B. aus geraden Reihen und Spalten bilden. Der Behälter 100 gemäß Figur 7 bildet ein Akupunkturnadelmagazin, aus dem die Akupunkturnadeln beispielsweise mit einem Betätigungsteil 40 freigegeben werden kann, wie es in den Figuren 1, 2 oder 4 gezeigt ist.

Die Zahl der im Behälterkörper 20 vorgesehenen Aufnahmekanäle 21 wird je nach den Dimensionen des zu lagernden Sterilguts und der konkreten Anwendung gewählt. Im Fall von Akupunkturnadeln kann sie beispielsweise 50 bis 100 oder mehr betragen.

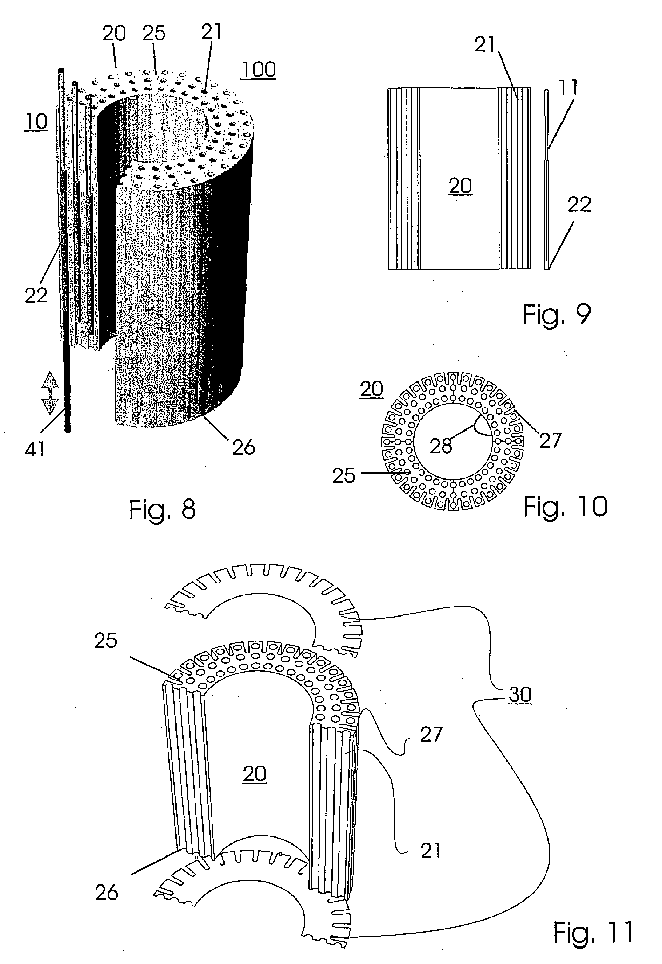

Eine abgewandelte Ausführungsform eines erfindungsgemäßen Behälters für eine Vielzahl von einzelnen Akupunkturnadeln ist in den Figuren 8 bis 11 illustriert. Figur 8 zeigt den Behälterkörper 20 in Form eines geraden Hohlzylinders, in dessen Mantel die Aufnahmekanäle 21 gebildet sind (Illustration ohne Abdeckung). Die Aufnahmekanäle 21 verlaufen gerade von der Stirnseite 25 zur Rückseite 26 parallel zur Zylinderachse. In jedem Aufnahmekanal 21 ist ein Ausstoßstempel 22 analog zu der Anwendung gemäß zu den Figuren 1 oder 2 oder ein Führungsröhrchen 13 analog zu der Anwendung gemäß Fig. 4 vorgesehen. Im linken Teil von Figur 8 ist das Verschieben des Ausstoßstempels 22 und der Akupunkturnadel 10 mit dem Betätigungsstift 41 illustriert.

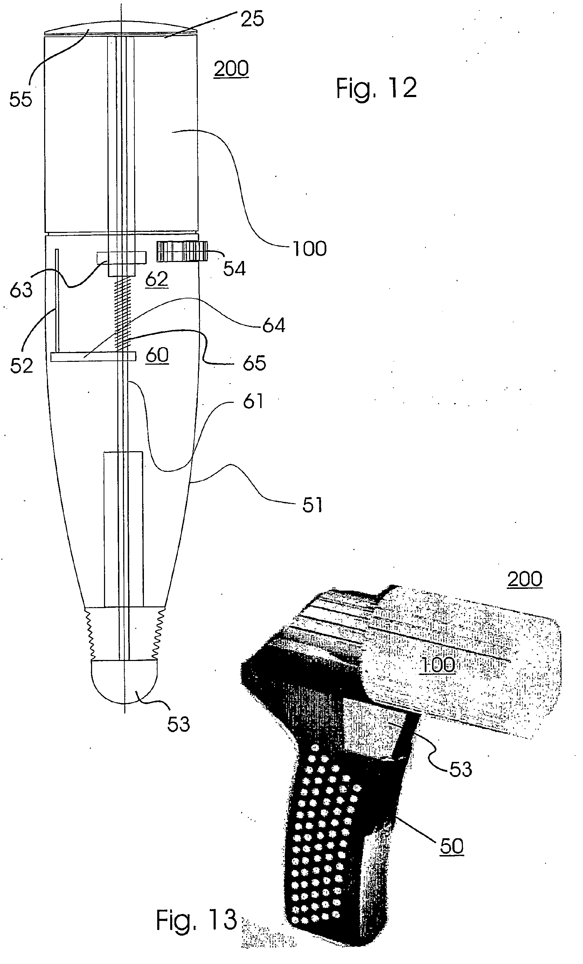

Figur 9 zeigt eine Schnittansicht des Behälterkörpers 20 mit den Aufnahmekanälen 21 und für Illustrationszwecke getrennt vom Behälterkörper 20 den Ausstoßstempel 22 mit der Akupunkturnadel 10, deren Nadelteil 12 in den Ausstoßstempel 22 hineinragt. Figur 10 zeigt eine Draufsicht auf die Stirnseite 25 des Behälterkörpers 20, in dessen Oberfläche Schlitze 27 eingearbeitet sein können. Die Schlitze 27 können für eine Materialersparnis und eine Gewichtsreduzierung von Vorteil sein und als Eingriff für eine Betätigungseinrichtung dienen. Figur 10 illustriert ferner einen Vorsprung 28, der im Innenraum des Behälterkörpers 20 vorgesehen sein kann und als Eingriff für eine Betätigungseinrichtung dienen kann (siehe Figuren 12 und 13).

Figur 11 illustriert die Anbringung der Abdeckung 30 auf den Stirn- und Rückseiten 25, 26 des Behälterkörpers 20 in geschnittener Perspektivansicht. Vorteilhafterweise werden auf beiden Seiten alle Aufnahmekanäle 21 jeweils mit einem durchgehenden Ring des Medical Paper oder einer entsprechenden Abdeckfolie verschlossen.

Der erfindungsgemäße Behälter 100 beispielsweise entsprechend den Figuren 7 bis 11 bildet gemäß einer bevorzugten Anwendung der Erfindung ein Magazin oder Spendermodul eines Dispensers für Sterilgut, insbesondere für Akupunkturnadeln. Zwei Ausführungsformen des erfindungsgemäßen Dispensers 200 sind beispielhaft in den Figuren 12 und 13 illustriert.

Der erfindungsgemäße Dispenser 200 umfasst gemäß Figur 12 einen Behälter 100 für Sterilgut und eine Betätigungseinrichtung 50, 60 zur manuellen Freigabe von Sterilgut aus dem Behälter 100. Der Behälter 100 ist beispielsweise als zylindrisches Magazin gemäß den Figuren 8 bis 11 zur Aufnahme von Akupunkturnadeln eingerichtet.

Die Betätigungseinrichtung 50, 60 umfasst ein Gehäuse 51, in dem ein Betätigungsstift 52 angeordnet ist, und eine Antriebseinrichtung 60 zum Antrieb des Betätigungsstiftes 52. Die Antriebseinrichtung 60 umfasst eine Vorschubstange 61, die von einer Betätigungstaste 53 bis zu einer Einstelleinrichtung 62 führt. Die Einstelleinrichtung 62 ist mit einer Rasteinrichtung 63 zur Ausrichtung des Behälters 100 und einem Träger 64 des Betätigungsstiftes 52 ausgestattet. Die genannten Teile wirken wie folgt zusammen.

Der Betätigungsstift 52 sitzt auf dem exzentrisch verstellbaren Träger 64, der mit einem Stellrad 54 in drei Positionen entsprechend den drei koaxialen Ringen von Aufnahmekanälen im Behälter 100 einstellbar ist (siehe Fig. 10). Bei Eindrücken der Betätigungstaste 53 vollzieht die Schubstange 61 eine Hubbewegung mit zwei Schritten. In dem ersten Schritt erfolgt eine Drehung des Behälters 100 zu einer neuen Position eines Aufnahmekanals. Hierzu wird die Schubbewegung der Vorschubstange 61 mit der Rasteinrichtung 63 in eine Drehbewegung entsprechend dem Rastermaß der Aufnahmekanäle im Behälter 100 umgewandelt. In dem zweiten Schritt wird der Betätigungsstift 52 verschoben, bis er in den Aufnahmekanal im Behälter 100 eindringt und in der oben beschriebenen Weise eine Akupunkturnadel 10 an der Stirnseite 25 des Behälters 100 freigibt. Unter der Wirkung der Feder 65 wird die Vorschubstange 61 bei Freigabe der Betätigungstaste 53 zurückgezogen. Das Bezugszeichen 55 verweist auf einen Schutzdeckel, der bei Gebrauch des Dispensers 200 abgenommen wird.

Abweichend von der in Figur 12 gezeigten axialen Ausrichtung der Vorschubstange 61 mit der Zylinderachse des Behälters 100 kann gemäß Figur 13 eine Achsenumlenkung vorgesehen sein. In diesem Fall ist die Antriebseinrichtung im Gehäuse 50 untergebracht, das einen Handgriff bildet. Die Verschiebung des Betätigungsstiftes erfolgt mit der Betätigungstaste 53. Alternativ kann der Dispenser 200 mit einer elektrisch betriebenen Antriebseinrichtung ausgestattet sein. Die Betätigungseinrichtung mit dem Behälter ist beispielsweise an einer Wand in einem Labor montiert und mit einem Stellmotor betätigbar.

Die in der vorstehenden Beschreibung, insbesondere den verschiedenen Ausführungsformen, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Behälter (100) zur Aufnahme von Sterilgut (10), mit einem Behälterkörper (20) mit mindestens einem durchgehenden Aufnahmekanal (21), der zumindest an einem Ende eine Abdeckung (30) aufweist, wobei
im Aufnahmekanal (21) ein Ausstoßstempel (22) verschiebbar angeordnet ist, dessen Stempellänge geringer als die Kanallänge des Aufnahmekanals (21) ist, so dass im Aufnahmekanal (21) zur Aufnahme des Sterilgutes (10) ein Hohlraum (23) gebildet wird, der mit der Abdeckung (30) nach außen verschlossen ist,
**dadurch gekennzeichnet, dass**
die Abdeckung (30) aus einem schichtförmigen Material (31) gebildet ist, das durch ein Vorschieben des Ausstoßstempels (22) mit dem Sterilgut (10) durchbrochen werden kann.

2. Behälter nach Anspruch 1, bei dem der Ausstoßstempel (22) die Form einer Hülse mit einer inneren Bohrung (24) besitzt.

3. Behälter nach Anspruch 1 oder 2, bei dem der Ausstoßstempel (22) im Aufnahmekanal (21) formschlüssig angeordnet ist.

4. Behälter nach mindestens einem der Ansprüche 1 bis 3, bei dem der Aufnahmekanal (21) an beiden Enden jeweils eine Abdeckung (31, 32) aufweist.

5. Behälter nach mindestens einem der Ansprüche 1 bis 4, bei dem die Abdeckung (30) schichtförmig aus einem mit dem Sterilgut (10) manuell zerstörbaren Material (31) gebildet ist.

6. Behälter nach Anspruch 5, bei dem die Abdeckung (30) aus Sterilpapier oder -folie (31) besteht.

7. Behälter nach mindestens einem der Ansprüche 2 bis 6, der zur Aufnahme von mindestens einer Akupunkturnadel (10) jeweils mit einem Nadelgriff (11) und einem Nadelteil (12) als Sterilgut eingerichtet ist, wobei der Aufnahmekanal (21) jeweils einen Kanalinnendurchmesser besitzt, der größer oder gleich dem Außendurchmesser des Nadelgriffes (11) ist.

8. Behälter nach den Ansprüchen 2 und 7, bei dem die innere Bohrung (24) des Ausstoßstempels (22) zur Aufnahme des Nadelteils (12) eingerichtet ist, so dass der Nadelteil (12) in die innere Bohrung (24) hineinragen kann.

9. Behälter nach Anspruch 8, bei dem der Durchmesser der inneren Bohrung (24) kleiner als der Außendurchmesser des Nadelgriffes (11) ist.

10. Behälter nach mindestens einem der vorhergehenden Ansprüche, bei dem in einer Dornaufnahme (29) des Aufnahmekanals (21) ein Dorn (70) angeordnet ist, mit dem die Abdeckung (30) geöffnet werden kann.

11. Behälter nach Anspruch 10, bei dem der Dorn an seinem zur Abdeckung (30) weisenden Ende eine abgeschrägte Schnittfläche (71) aufweist.

12. Behälter nach Anspruch 10 oder 11, bei dem der Dorn (70) einen radialen Vorsprung (72) aufweist, mit dem der Dorn (70) bei geöffneter Abdeckung (30) im Aufnahmekanal (21) zurückgehalten werden kann.

13. Behälter nach mindestens einem der Ansprüche 10 bis 12, bei dem der Dorn (70) einen axial verlaufenden Hohlkanal aufweist, dessen Innendurchmesser größer als der Außendurchmesser des zur Aufnahme im Behälter (100) vorgesehenen Sterilgutes (10) ist.

14. Behälter nach Anspruch 13, bei dem am rückseitigen Ende des Dorn (70) eine Hülse (73) vorgesehen ist, deren Innendurchmesser so gewählt ist, dass ein Anschlag zur Übertragung einer Vorschubbewegung des Sterilguts (10) auf den Dorn (70) gebildet wird, und
bei dem der Innendurchmesser der Hülse (73) und/oder des gesamten Dorns (70) vergrößerbar ist, so dass das Sterilgut (10) durch den hohlen Dorn (70) geschoben werden kann,
wobei die Hülse (73) und/oder der gesamte Dorn (70) aus einem federnden Material gebildet ist, das in einem ersten Teilbereich des Aufnahmekanals (21) vorgespannt ist, so dass der Anschlag gebildet wird, und das in der benachbarten Dornaufnahme (29) des Aufnahmekanals (21) mit einem vergrößerten Durchmesser entspannt ist, so dass sich der Innendurchmesser der Hülse (73) vergrößert.

15. Behälter nach mindestens einem der vorhergehenden Ansprüche, bei dem der Ausstoßstempel (22) ein Führungsröhrchen (13) für eine Akupunkturnadel (10) umfasst.

16. Behälter nach mindestens einem der vorhergehenden Ansprüche, der mit einem Betätigungsteil (40) zur Verschiebung des Ausstoßstempels (22) im Aufnahmekanal (21) ausgestattet ist.

17. Behälter nach Anspruch 16, bei dem das Betätigungsteil (40) mindestens einen Betätigungsstift (41, 41A, 41B) aufweist, dessen Durchmesser kleiner oder gleich dem Kanalinnendurchmesser des Aufnahmekanals (21) ist.

18. Behälter nach mindestens einem der Ansprüche 1 bis 17, bei dem im Behälterkörper (20) eine Vielzahl von Aufnahmekanälen (21) gebildet sind.

19. Behälter nach Anspruch 18, bei dem die Aufnahmekanäle (21) von einer Stirnseite (25) zur einer Rückseite (26) des Behälterkörpers (20) parallel verlaufen.

20. Behälter nach Anspruch 19, bei dem der Behälterkörper (20) die Form eines Kreiszylinders zwischen den Stirn- und Rückseiten (25, 26) besitzt.

21. Behälter nach Anspruch 20, bei dem die Aufnahmekanäle (21) im Behälterkörper (20) so angeordnet sind, dass ihre Schnittpunkte mit einer Querschnittsfläche des Behälterkörpers (20) zwei oder mehr koaxiale Kreise bilden.

22. Behälter nach Anspruch 21, bei dem die Aufnahmekanäle (21) im Behälterkörper (20) radial fluchtend angeordnet sind.

23. Behälter nach Anspruch 19, bei dem der Behälterkörper (20) die Form eines Quaders zwischen den Stirn- und Rück- seiten (25, 26) besitzt.

24. Behälter nach mindestens einem der Ansprüche 18 bis 23, bei dem auf den Stirn- und Rückseiten (25, 26) die Abdeckung (30) für alle Aufnahmekanäle (21) gemeinsam jeweils aus einem schichtförmigen Material (31, 32) gebildet ist.

25. Dispenser (200) für Sterilgut, der eine Betätigungseinrichtung (50, 60) und einen Behälter (100) nach mindestens einem der Ansprüche 1 bis 19 aufweist.

26. Dispenser nach Anspruch 25, bei dem die Betätigungseinrichtung (50, 60) ein Gehäuse (51) aufweist, an dem der Behälter (100) angebracht ist, wobei im Gehäuse (51) ein Betätigungsstift (52) beweglich angeordnet ist.

27. Dispenser nach Anspruch 26, bei dem der Betätigungsstift (52) mit einer manuell betätigbaren Antriebseinrichtung (60) beweglich ist.

28. Dispenser nach Anspruch 27, bei dem die Antriebseinrichtung (60) eine federnd im Gehäuse (51) angeordnete Vorschubstange (61) aufweist, an der der Betätigungsstift (52) befestigt ist.

29. Dispenser nach Anspruch 28, bei dem der Betätigungsstift (52) an der Vorschubstange (61) exzentrisch so befestigt ist, dass verschiedene radiale Betriebspositionen des Betätigungsstifts (52) einstellbar sind.

30. Dispenser nach mindestens einem der Ansprüche 27 bis 29, bei dem die Antriebseinrichtung (60) eine im Gehäuse (51) angeordnete Einstelleinrichtung (62) zur Ausrichtung des Behälters (100) relativ zur Betriebsposition des Betätigungsstifts (52) aufweist.

31. Dispenser nach Anspruch 30 mit Rückbezug auf Anspruch 28, bei dem die Einstelleinrichtung (62) eine mit der Vorschubstange (61) verbundene Rasteinrichtung (63) aufweist, mit der der Behälter (100) vor jeder Betätigung des Betätigungsstifts (52) um eine Wegstrecke verstellbar ist, die dem Abstand zwischen zwei Aufnahmekanälen (21) im Behälterkörper (21) entspricht.

32. Dispenser nach mindestens einem der Ansprüche 26 bis 31 mit Rückbezug auf Anspruch 19, bei dem das Gehäuse (50) als Handgriff gebildet ist, der sich parallel zur Ausrichtung der Aufnahmekanälen (21) im Behälterkörper (21) erstreckt.

33. Dispenser nach mindestens einem der Ansprüche 26 bis 31 mit Rückbezug auf Anspruch 19, bei dem das Gehäuse (50) als Handgriff gebildet ist, der sich senkrecht zur Ausrichtung der Aufnahmekanälen (21) im Behälterkörper (21) erstreckt.

34. Dispenser nach Anspruch 26, bei dem der Betätigungsstift (52) mit einer elektrisch betriebenen Antriebseinrichtung beweglich ist.

35. Verfahren zur Bereitstellung von Sterilgut mit einem Dispenser (200) nach mindestens einem der Ansprüche 25 bis 34, wobei jeweils ein Teil des Sterilguts aus dem Behälter (100) geschoben wird, indem mit der Betätigungseinrichtung (50, 60) im entsprechenden Aufnahmekanal (22) der Ausstoßstempel (22) verschoben wird, bis das Teil des Sterilguts (10) die Abdeckung (30) durchbricht und zur weiteren Verwendung aus dem Behälterkörper (20) ragt.

36. Verfahren nach Anspruch 35, wobei jeweils als Sterilgut jeweils eine Akupunkturnadel (10) mit einem Nadelgriff (11) und einem Nadelteil (12) aus dem Behälter (100) geschoben wird, bis der Nadelgriff (11) die Abdeckung (30) durchbricht und aus dem Behälterkörper (20) ragt.

## Claims

1. A container (100) for accommodating sterile material (10), with a container body (20) with at least one through receiving channel (21), which comprises a cover (30) on at least one end, wherein
a pushing ram (22) being arranged displaceably in the receiving channel (21), the ram length of which is smaller than the channel length of the receiving channel (21), such that a cavity (23) is formed in the receiving channel (21) for accommodating the sterile material (10), which cavity is closed relative to the outside by the cover (30),
**characterised in that**
the cover (30) is made from a layer-shaped material (31), which may be perforated by the sterile material (10) through advance of the pushing ram (22).

2. The container according to claim 1, in which the pushing ram (22) takes the form of a sleeve with an internal bore (24).

3. The container according to claim 1 or claim 2, in which the pushing ram (22) is arranged in form-fitting manner in the receiving channel (21).

4. The container according to at least one of claims 1 to 3, in which the receiving channel (21) comprises a cover (31, 32) at each end.

5. The container according to at least one of claims 1 to 4, in which the cover (30) is formed in layer form from a material (31) which may be destroyed manually with the sterile material (10).

6. The container according to claim 5, in which the cover (30) consists of sterile paper or sterile foil (31).

7. The container according to at least one of claims 2 to 6, which is designed to accommodate as sterile material at least one acupuncture needle (10) each having a needle handle (11) and a needle portion (12), wherein the receiving channel (21) in each case has an internal channel diameter which is greater than or equal to the external diameter of the needle handle (11).

8. The container according to claims 2 and 7, in which the internal bore (24) of the pushing ram (22) is designed to accommodate the needle portion (12), such that the needle portion (12) may protrude into the internal bore (24).

9. The container according to claim 8, in which the diameter of the internal bore (24) is less than the external diameter of the needle handle (11).

10. The container according to at least one of the preceding claims, in which a mandrel (70) is arranged in a mandrel receptacle (29) of the receiving channel (21), with which mandrel the cover (30) may be opened.

11. The container according to claim 10, in which the mandrel comprises a bevelled cut face (71) at its end directed towards the cover (30).

12. The container according to claim 10 or claim 11, in which the mandrel (70) comprises a radial projection (72), with which the mandrel (70) may be retained in the receiving channel (21) when the cover (30) is opened.

13. The container according to at least one of claims 10 to 12, in which the mandrel (70) comprises an axially extending hollow channel, the internal diameter of which is greater than the external diameter of the sterile material (10) intended to be accommodated in the container (100).

14. The container according to claim 13, in which a sleeve (73) is provided at the rear end of the mandrel (70), the internal diameter of which sleeve is selected such that a limit stop is formed for transmitting a feed movement of the sterile material (10) to the mandrel (70), and
in which the internal diameter of the sleeve (73) and/or of the entire mandrel (70) may be enlarged, such that the sterile material (10) may be pushed through the hollow mandrel (70),
wherein the sleeve (73) and/or the entire mandrel (70) is made of a resilient material, which is pretensioned in a first sub-region of the receiving channel (21), such that the limit stop is formed, and which is relieved of said pretension in the adjacent mandrel receptacle (29) of the receiving channel (21) with a larger diameter, such that the internal diameter of the sleeve (73) becomes larger.

15. The container according to at least one of the preceding claims, in which the pushing ram (22) comprises a guide tube (13) for an acupuncture needle (10).

16. The container according to at least one of the preceding claims, which is provided with an actuating element (40) for displacing the pushing ram (22) in the receiving channel (21).

17. The container according to claim 16, in which the actuating element (40) comprises at least one actuating pin (41, 41A, 41 B), the diameter of which is less than or equal to the internal channel diameter of the receiving channel (21).

18. The container according to at least one of claims 1 to 17, in which a plurality of receiving channels (21) are formed in the container body (20).

19. The container according to claim 18, in which the receiving channels (21) extend in parallel from the front face (25) to the rear face (26) of the container body (20).

20. The container according to claim 19, in which the container body (20) is circular-cylindrical in shape between the front and rear faces (25, 26).

21. The container according to claim 20, in which the receiving channels (21) are arranged in the container body (20) in such a way that their points of intersection with a cross section of the container body (20) form two or more coaxial circles.

22. The container according to claim 21, in which the receiving channels (21) are arranged in a radially in-line arrangement in the container body (20).

23. The container according to claim 19, in which the container body (20) is cuboid in shape between the front and rear faces (25, 26).

24. The container according to at least one of claims 18 to 23, in which on the front and rear faces (25, 26) the cover (30) is in each case formed jointly for all the receiving channels (21) from a layer-shaped material (31, 32).

25. A dispenser (200) for sterile material, which comprises an actuator (50, 60) and a container (100) according to at least one of claims 1 to 19.

26. The dispenser according to claim 25, in which the actuator (50, 60) comprises a housing (51), on which the container (100) is mounted, an actuating pin (52) being arranged movably in the housing (51).

27. The dispenser according to claim 26, in which the actuating pin (52) is movable by a manually actuatable drive device (60).

28. The dispenser according to claim 27, in which the drive device (60) comprises a feed rod (61) arranged in spring-mounted manner in the housing (51), to which feed rod (61) the actuating pin (52) is attached.

29. The dispenser according to claim 28, in which the actuating pin (52) is attached eccentrically to the feed rod (61) in such a way that various radial operating positions of the actuating pin (52) may be set.

30. The dispenser according to at least one of claims 27 to 29, in which the drive device (60) comprises an adjusting means (62) arranged in the housing (51) for orienting the container (100) relative to the operating position of the actuating pin (52).

31. The dispenser according to claim 30 with reference to claim 28, in which the adjusting means (62) comprises a locking means (63) connected to the feed rod (61), with which the container (100) may be adjusted prior to each actuation of the actuating pin (52) by a distance which corresponds to the spacing between two receiving channels (21) in the container body (20).

32. The dispenser according to at least one of claims 26 to 31 with reference to claim 19, in which the housing (50) takes the form of a hand grip which extends parallel to the orientation of the receiving channels (21) in the container body (20).

33. The dispenser according to at least one of claims 26 to 31 with reference to claim 19, in which the housing (50) takes the form of a hand grip which extends perpendicular to the orientation of the receiving channels (21) in the container body (20).

34. The dispenser according to claim 26, in which the actuating pin (52) is movable by an electrically operated drive device.

35. A method of providing sterile material with a dispenser (200) according to at least one of claims 25 to 34, wherein in each case a part of the sterile material is pushed out of the container (100) by displacing the pushing ram (22) with the actuating device (50, 60) in the appropriate receiving channel (21) until the part of the sterile material (10) perforates the cover (30) and projects out of the container body (20) for further use.

36. The method according to claim 35, wherein as sterile material in each case one acupuncture needle (10) with a needle handle (11) and a needle portion (12) is pushed out of the container (100) until the needle handle (11) perforates the cover (30) and projects out of the container body (20).

## Revendications

1. Conteneur (100) destiné à recevoir un article stérile (10), comportant un corps de conteneur (20) avec au moins un canal de réception (21) traversant, lequel présente un couvercle (30) au moins à une extrémité, où
un tampon d'expulsion (22) est disposé de manière à être mobile dans le canal de réception (21), la longueur de ce tampon étant inférieure à la longueur du canal de réception (21), de manière à former une cavité (23) pour le logement de l'article stérile (10) dans le canal de réception (21), laquelle est obturée par le couvercle (30) par rapport à l'extérieur,
**caractérisé**
**en ce que** le couvercle (30) est constitué d'un matériau (31) en couche qui peut être percé par une poussée du tampon d'expulsion (22) par l'article stérile (10).

2. Conteneur selon la revendication 1, où le tampon d'expulsion (22) a la forme d'une douille avec un alésage intérieur (24).

3. Conteneur selon la revendication 1 ou la revendication 2, où le tampon d'expulsion (22) est disposé avec correspondance de forme dans le canal de réception (21).

4. Conteneur selon au moins une des revendications 1 à 3, où le canal de réception (21) comporte un couvercle (31, 32) à chacune de ses deux extrémités.

5. Conteneur selon au moins une des revendications 1 à 4, où le couvercle (30) est constitué d'un matériau (31) en couche manuellement destructible au moyen de l'article stérile (10).

6. Conteneur selon la revendication 5, où le couvercle (30) est constitué d'un papier ou d'un film stérile (31).

7. Conteneur selon au moins une des revendications 2 à 6, prévu pour la réception d'au moins une aiguille d'acupuncture (10) pourvue d'une poignée d'aiguille (11) et d'une partie d'aiguille (12) en tant qu'article stérile, le canal de réception (21) présentant un diamètre intérieur de canal égal ou supérieur au diamètre extérieur de la poignée d'aiguille (11).

8. Conteneur selon les revendications 2 et 7, où l'alésage intérieur (24) du tampon d'expulsion (22) est prévu pour la réception de la partie d'aiguille (12), de manière à permettre à la partie d'aiguille (12) de pénétrer dans l'alésage intérieur (24).

9. Conteneur selon la revendication 8, où le diamètre de l'alésage intérieur (24) est inférieur au diamètre extérieur de la poignée d'aiguille (11).

10. Conteneur selon au moins une des revendications précédentes, où une broche (70) est disposée dans un logement de broche (29) du canal de réception (21), au moyen de laquelle le couvercle (30) peut être ouvert.

11. Conteneur selon la revendication 10, où la broche présente une face de coupe (71) biseautée à son extrémité dirigée vers le couvercle (30).

12. Conteneur selon la revendication 10 ou la revendication 11, où la broche (70) présente une saillie (72) radiale permettant de retenir la broche (70) dans le canal de réception (21) lorsque le couvercle (30) est ouvert.

13. Conteneur selon au moins une des revendications 10 à 12, où la broche (70) comporte un canal creux à extension axiale, dont le diamètre intérieur est supérieur au diamètre extérieur de l'article stérile (10) destiné à être logé dans le conteneur (100).

14. Conteneur selon la revendication 13, où une douille (73) est prévue à l'extrémité arrière de la broche (70), dont le diamètre intérieur est sélectionné de manière à former une butée pour transmettre un mouvement de poussée de l'article stérile (10) à la broche (70), et
où le diamètre intérieur de la douille (73) et/ou de l'ensemble de la broche (70) peut être agrandi, de telle sorte que l'article stérile (10) peut être poussé par la broche creuse (70),
la douille (73) et/ou l'ensemble de la broche (70) étant constituées d'un matériau élastique pré-contraint dans une première partie du canal de réception (21), de manière à former la butée, et détendu avec un diamètre agrandi dans le logement de broche (29) adjacent du canal de réception (21), de manière à agrandir le diamètre intérieur de la douille (73).

15. Conteneur selon au moins une des revendications précédentes, où le tampon d'expulsion (22) comprend une tubulure de guidage (13) pour une aiguille d'acupuncture (10).

16. Conteneur selon au moins une des revendications précédentes, pourvu d'une pièce d'actionnement (40) pour la poussée du tampon d'expulsion (22) dans le canal de réception (21).

17. Conteneur selon la revendication 16, où la pièce d'actionnement (40) comporte au moins une tige d'actionnement (41, 41A, 41B), dont le diamètre est inférieur ou égal au diamètre intérieur du canal de réception (21).

18. Conteneur selon au moins une des revendications 1 à 17, où une pluralité de canaux de réception (21) sont formés dans le corps de conteneur (20).

19. Conteneur selon la revendication 18, où les canaux de réception (21) s'étendent parallèlement d'une face avant (25) vers une face arrière (26) du corps de conteneur (20).

20. Conteneur selon la revendication 19, où le corps de conteneur (20) présente la forme d'un cylindre circulaire entre les faces avant et arrière (25, 26).

21. Conteneur selon la revendication 20, où les canaux de réception (21) sont disposés dans le corps de conteneur (20) de telle sorte que leurs points d'intersection forment deux cercles coaxiaux ou plus avec une surface de section du corps de conteneur (20).

22. Conteneur selon la revendication 21, où les canaux de réception (21) sont disposés radialement alignés dans le corps de conteneur (20).

23. Conteneur selon la revendication 19, où le corps de conteneur (20) présente la forme d'un parallélépipède droit entre les faces avant et arrière (25, 26).

24. Conteneur selon au moins une des revendications 18 à 23, où le couvercle (30) pour l'ensemble des canaux de réception (21) sur les faces avant et arrière (25, 26) est constitué d'un matériau (31, 32) en couche.

25. Distributeur (200) pour articles stériles, comportant un dispositif d'actionnement (50, 60) et un conteneur (100) selon au moins une des revendications 1 à 19.

26. Distributeur selon la revendication 25, où le dispositif d'actionnement (50, 60) comporte un boîtier (51) contre lequel le conteneur (100) est appliqué, une tige d'actionnement (52) étant disposée de manière à être mobile dans le boîtier (51).

27. Distributeur selon la revendication 26, où la tige d'actionnement (52) est mobile au moyen d'un dispositif d'entraînement (60) actionnable manuellement.,

28. Distributeur selon la revendication 27, où le dispositif d'entraînement (60) comporte une tringle de poussée (61) montée sur ressort dans le boîtier (51), contre laquelle est fixée la tige d'actionnement (52).

29. Distributeur selon la revendication 28, où la tige d'actionnement (52) est fixée excentriquement contre la tringle de poussée (61), de manière à rendre réglables différentes positions radiales de fonctionnement de la tige d'actionnement (52).

30. Distributeur selon au moins une des revendications 27 à 29, où le dispositif d'entraînement (60) comporte un dispositif de réglage (62) monté dans le boîtier (51) pour l'ajustement du conteneur (100) par rapport à la position de fonctionnement de la tige d'actionnement (52).

31. Distributeur selon la revendication 30 en référence à la revendication 28, où le dispositif de réglage (62) comporte un dispositif d'enclenchement (63) raccordé à la tringle de poussée (61), au moyen duquel le conteneur (100) est déplaçable d'une course correspondant à l'espacement entre deux canaux de réception (21) dans le corps de conteneur (20), avant chaque actionnement de la tige d'actionnement (52).

32. Distributeur selon au moins une des revendications 26 à 31, où le boîtier (50) est formé comme une poignée qui s'étend parallèlement à la direction des canaux de réception (21) dans le corps de conteneur (20).

33. Distributeur selon au moins une des revendications 26 à 31, où le boîtier (50) est formé comme une poignée qui s'étend perpendiculairement à la direction des canaux de réception (21) dans le corps de conteneur (20).

34. Distributeur selon la revendication 26, où la tige d'actionnement (52) est mobile au moyen d'un dispositif d'entraînement à commande électrique.

35. Procédé pour la mise à disposition d'un article stérile au moyen d'un distributeur (200) selon au moins une des revendications 25 à 34, où une pièce d'article stérile est sortie du conteneur (100) par poussée du tampon d'expulsion (22) au moyen du dispositif d'actionnement (50, 60) dans le canal de réception (22) correspondant, jusqu'à ce que la pièce d'article stérile (10) perce le couvercle (30) et sorte du corps de conteneur (20) pour utilisation.

36. Procédé selon la revendication 35, où une aiguille d'acupuncture (10) est poussée du conteneur (100) en tant qu'article stérile, avec une poignée d'aiguille (11) et une partie d'aiguille (12), jusqu'à ce que la poignée d'aiguille (11) perce le couvercle (30) et fasse saillie hors du corps de conteneur (20).
